# EUROPÄISCHE PATENTANMELDUNG

(11) **EP 4 786 661 A1**
(43) Veröffentlichungstag der Anmeldung: **05.08.2026**
(21) Anmeldenummer: 26154369.8
(22) Anmeldetag: 27.01.2026
(51) Int. Cl.: D01H 13/22, G01N 33/36

(54) **VERFAHREN ZUR BESTIMMUNG EINES EIGENSCHAFTSWERTS EINES GESPONNENEN KERNGARNS, VERFAHREN ZUM BETRIEB EINES SPINNSTELLENSYSTEMS, VORRICHTUNG ZUR DATENVERARBEITUNG, FADENSENSOREINRICHTUNG, SPINNSTELLENSYSTEM UND COMPUTERPROGRAMM**

(30) Priorität: 03.02.2025 LU 509836
(71) Anmelder: Saurer Spinning Solutions GmbH & Co. KG, 52531 Übach-Palenberg (DE)
(72) Erfinder: Spitzer, Michael, 52156 Monschau-Kalterherberg (DE); Küppers, Dr. Simon, 70439 Stuttgart (DE)
(74) Vertreter: Morgenthum-Neurode, Mirko

(57) **Zusammenfassung**

Die Erfindung betrifft ein Verfahren zur Bestimmung eines Eigenschaftswerts eines gesponnenen Kerngarns, wobei auf Basis einer Auswertung mindestens eines Erfassungswerts, der vorzugsweise für einen Querschnitt des Kerngarns spezifisch ist, der Eigenschaftswert bestimmt wird. Die Erfindung betrifft ferner ein Verfahren zum Betrieb eines Spinnstellensystems, eine Vorrichtung zur Datenverarbeitung, eine Fadensensoreinrichtung, ein Spinnstellensystem und ein Computerprogramm.

## Beschreibung

Die vorliegende Erfindung betrifft ein Verfahren zur Bestimmung eines Eigenschaftswerts eines gesponnenen Kerngarns, ein Verfahren zum Betrieb eines Spinnstellensystems, eine Vorrichtung zur Datenverarbeitung, eine Fadensensoreinrichtung, ein Spinnstellensystem und ein Computerprogramm.

### Stand der Technik

Bei der Herstellung von gesponnenem Kerngarn ist es bevorzugt Eigenschaften des Kerngarns zu prüfen, um die Qualität des gesponnenen Kerngarns während der Herstellung des Kerngarns sicherzustellen.

Die Druckschrift EP 1 051 595 B1 offenbart ein Verfahren und eine Vorrichtung zum Bestimmen einer bewegten linearen Textilformation.

Die Druckschrift EP 1 889 956 B1 offenbart ein Verfahren und eine Anordnung zur Detektion der Anwesenheit eines Kernfilaments in einem Kerngarn beim Kerngarnspinnen.

Nachteilig an den Lösungen aus dem Stand der Technik ist insbesondere ihre Komplexität.

### Offenbarung der Erfindung

Eine Aufgabe der Erfindung ist es, eine alternative oder bessere technische Lösung zur Prüfung von gesponnenem Kerngarn bereitzustellen, die vorzugsweise die Nachteile aus dem Stand der Technik überwindet.

Die voranstehende Aufgabe wird gelöst durch ein Verfahren zur Bestimmung eines Eigenschaftswerts eines gesponnenen Kerngarns, ein Verfahren zum Betrieb eines Spinnstellensystems, eine Vorrichtung zur Datenverarbeitung, eine Fadensensoreinrichtung, ein Spinnstellensystem sowie ein Computerprogramm mit den Merkmalen der entsprechenden unabhängigen Ansprüche. Weitere Merkmale und Details der Erfindung ergeben sich aus den jeweiligen Unteransprüchen, der Beschreibung und den Zeichnungen. Dabei gelten Merkmale und Details, die im Zusammenhang mit dem erfindungsgemäßen Verfahren zur Bestimmung eines Eigenschaftswerts beschrieben sind, selbstverständlich auch im Zusammenhang mit dem erfindungsgemäßen Verfahren zum Betrieb eines Spinnstellensystems, der erfindungsgemäßen Vorrichtung zur Datenverarbeitung, der erfindungsgemäßen Fadensensoreinrichtung, dem erfindungsgemäßen Spinnstellensystem und dem erfindungsgemäßen Computerprogramm und jeweils umgekehrt, so dass bezüglich der Offenbarung zu den einzelnen Erfindungsaspekten stets wechselseitig Bezug genommen wird bzw. werden kann.

Das erfindungsgemäße Verfahren zur Bestimmung eines Eigenschaftswerts eines gesponnenen Kerngarns, wobei das Kerngarn einen Kernfaden und Mantelfasern umfasst, die den Kernfaden umgeben, weist die nachfolgenden Schritte auf:
- Empfangen von mindestens einem Erfassungswert, wobei der mindestens eine Erfassungswert vorzugsweise für einen Querschnitt des Kerngarns spezifisch ist,
- Auswerten des mindestens einen Erfassungswerts, wobei das Auswerten einen Vergleich zwischen dem mindestens einen Erfassungswert und mindestens einem Soll-Wert umfasst,
- Bestimmen des Eigenschaftswerts des Kerngarns auf Basis der Auswertung, insbesondere eines akzeptablen Eigenschaftswerts, wenn der Erfassungswert einem Soll-Wert entspricht und/oder eines inakzeptablen Eigenschaftswerts, wenn der Erfassungswert vom Soll-Wert abweicht.

Somit kann insbesondere mithilfe einer Sensorik der Ist-Zustand und vorzugsweise ein geometrisches Bild vom Ist-Zustand des Garnes ermittelt werden. Hierdurch wird eine alternative oder bessere technische Lösung zur Prüfung von Fäden, insbesondere Kerngarn, bereitgestellt, die insbesondere die Nachteile aus dem Stand der Technik überwindet. Mit anderen Worten handelt es sich bei dem erfindungsgemäßen Verfahren vorzugsweise um eine Untersuchung oder Prüfung des Kerngarns, insbesondere des gesponnenen Kerngarns. Der Erfassungswert bzw. der Ist-Zustand kann ferner mit dem Soll-Wert verglichen werden. Dabei kann eine Mittenlage des Kernfadens und die Abdeckung mit Mantelfasern verglichen werden. Ferner können, ggf. auch automatisiert, Maßnahmen zur Korrektur eingeleitet werden, falls sich die Abweichung vom Soll-Wert ergibt.

Nachfolgend werden einige Maßnahmen beispielhaft angeführt, welche eine Veränderung eines Parameters umfassen, um auf eine Abweichung vom Soll-Wert zu reagieren.

Falls sich der Kernfaden außerhalb der Mitte des Garnes befindet, kann ggf. die Korrektur durch eine Geschwindigkeitsänderung der Kernfadenzuführung erfolgen. Hierzu können die Geschwindigkeiten vorgelagerter Kernfaden-Förderwerke angepasst werden.

Befindet sich bspw. zu wenig Mantelfaser im Kerngarn, kann dies zu einer Erhöhung der Einspeisung verändert werden. Hierzu kann bspw. eine Erhöhung einer Zuführgeschwindigkeit von Fasermaterial und z. B. einer Auflösewalzen-Drehzahl vorgesehen sein. Auch kann eine Reduzierung der Drehzahl des garnbildenden Prozesses vorgenommen werden.

Es ist möglich, dass die erfindungsgemäßen Schritte, vorzugsweise zur sensorischen Beobachtung des Kerngarns, wiederholt ausgeführt werden. Die Beobachtung kann ggf. auch weiterhin durchgeführt werden, nachdem die voranstehenden Parameter verändert wurden. Befindet sich dann der Kernfaden ggf. zentrisch und ist eine ausreichende Abdeckung vorhanden, kann der Produktionsprozess weiterlaufen. Sind die Anforderungen hingegen nicht erfüllt, können ggf. erneut Parameter verändert werden.

Bei dem Kerngarn handelt es sich vorzugsweise um einen Spinnfaden. Das Kerngarn weist vorzugsweise einen Kernfaden auf, der von einer Schicht von Mantelfasern, insbesondere direkt, umgeben ist. Bei Mantelfasern handelt es sich vorzugsweise um Fasermaterial.

Es ist bevorzugt, wenn der Erfassungswert mittels eines Sensors, mehrerer Sensoren oder einer Fadensensoreinrichtung, insbesondere direkt oder indirekt, erfasst wurde. Darüber hinaus ist es denkbar, dass das Erfassen des mindestens einen Erfassungswerts, insbesondere mittels eines Sensors, mehrerer Sensoren oder einer Fadensensoreinrichtung, Teil des erfindungsgemäßen Verfahrens zur Bestimmung eines Eigenschaftswerts eines Kerngarns ist. Bei dem Sensor oder den Sensoren kann es sich um kapazitive Sensoren und/oder optische Sensoren und/oder Bildsensoren handeln.

Bei dem Erfassungswert handelt es sich vorzugsweise um ein Bild, insbesondere ein geometrisches Bild des Kerngarns, insbesondere des Querschnitts und/oder der Oberfläche des Kerngarns.

Darüber hinaus kann es sich bei dem Erfassungswert um einen Messwert, also insbesondere einen gemessenen, vorzugsweise direkt oder indirekt gemessenen Wert, und/oder einen Sensorwert, insbesondere einen mittels eines Sensors gemessenen, vorzugsweise direkt oder indirekt gemessenen Wert handeln.

Der Erfassungswert kann zum Beispiel in Form von Daten, insbesondere digital oder analog, vorliegen. Der Eigenschaftswert entspricht vorzugsweise einem Ist-Eigenschaftswert des Kerngarns.

Bei dem Soll-Wert handelt es sich vorzugsweise um einen Soll-Erfassungswert. Der Soll-Wert oder der Soll-Erfassungswert ist vorzugsweise spezifisch für einen Soll-Querschnitt des Kerngarns.

Das Empfangen kann darüber hinaus kabelgebunden oder kabellos, insbesondere mittels Funkübertragung, erfolgen. Der Erfassungswert kann in verschlüsselter Form empfangen werden. Hierbei ist es zweckmäßig, wenn der verschlüsselte Erfassungswert vor dem Auswerten entschlüsselt wird. Durch die Verschlüsselung kann die Betriebssicherheit sichergestellt werden.

Das Empfangen kann mittels einer Empfangseinheit durchgeführt werden. Das Auswerten kann hingegen mittels einer Auswerteeinheit ausgeführt werden. Die Empfangseinheit und die Auswerteeinheit können in eine Steuereinheit, insbesondere eines Spinnstellensystems, integriert sein.

Vorzugsweise wird das erfindungsgemäße Verfahren in Echtzeit und/oder während der Herstellung des Kerngarns und, insbesondere lokal gesehen, nach einer Spinnstelle oder Spinnstellenvorrichtung durchgeführt.

Zur Dokumentation kann vorgesehen sein, dass der mindestens eine Erfassungswert oder der Eigenschaftswert abgespeichert wird. Ein derartiger Erfassungswert oder Eigenschaftswert kann dem Kerngarn zugeordnet sein oder zugeordnet werden.

Ferner können mehrere Erfassungswerte empfangen werden, die jeweils für verschiedene Abschnitte des Kernfadens charakteristisch sind. Hierbei ist es bevorzugt, wenn die entsprechenden Erfassungswerte nacheinander, insbesondere in zeitlichem Abstand zueinander, in der Reihenfolge ihrer Erfassung empfangen werden.

Es ist bevorzugt, wenn sich der mindestens eine Erfassungswert auf einen Abschnitt, insbesondere Längenabschnitt, des Kerngarns bezieht. Ferner ist es bevorzugt, wenn das Verfahren für mehrere aneinandergrenzende oder zueinander beabstandete oder alle Abschnitte, insbesondere Längenabschnitte, des Kerngarns durchgeführt wird. Somit ist eine stichprobenartige Untersuchung des Kernfadens oder eine kontinuierliche Untersuchung des Kernfadens realisierbar.

Es ist denkbar, dass das erfindungsgemäße Verfahren durch eine Initialisierung eines Spinnvorgangs oder eines Spinnverfahrens initialisiert wird. Hierbei ist es zweckmäßig, wenn das erfindungsgemäße Verfahren beendet oder unterbrochen wird, wenn der Spinnvorgang oder das Spinnverfahren beendet oder unterbrochen wird. Mit dem Spinnverfahren oder dem Spinnvorgang ist vorzugsweise eine Herstellung eines Spinnfadens, also des Kerngarns, gemeint.

Darüber hinaus ist es bevorzugt, wenn der mindestens eine Erfassungswert für mindestens einen der folgenden Werte spezifisch ist:
- eine Querschnittsdicke des Kerngarns,
- eine aus den Mantelfasern gebildete Mantelfaserschicht des Kerngarns, insbesondere eine Querschnittsdicke der Mantelfaserschicht und/oder eine Abdeckung des Kernfadens mit Mantelfasern,
- eine Querschnittsdicke des Kernfadens des Kerngarns,
- eine Lage des Kernfadens innerhalb des Querschnitts des Kerngarns,
und wobei der Soll-Wert vorzugsweise für mindestens einen zum Erfassungswert entsprechenden Soll-Wert spezifisch ist, insbesondere für einen der folgenden Soll-Werte:
- eine Soll-Querschnittsdicke des Kerngarns,
- eine Soll-Mantelfaserschicht des Kerngarns, insbesondere eine Soll-Querschnittsdicke der Mantelfaserschicht, und/oder eine Soll-Abdeckung des Kernfadens mit Mantelfasern,
- eine Soll-Querschnittsdicke des Kernfadens des Kerngarns,
- eine Soll-Lage des Kernfadens innerhalb des Querschnitts des Kerngarns.

Die Querschnittsdicken, insbesondere Querschnittsstärken, sind vorzugsweise senkrecht zu einer, insbesondere geradlinigen, Erstreckungsrichtung und/oder Förderrichtung des Kerngarns messbar.

Bei den Soll-Querschnittsdicken handelt es sich vorzugsweise um Mindest-Querschnittsdicken.

Bei der Lage handelt es sich vorzugsweise um eine Relativ-Lage, insbesondere eine Lage relativ zur Außenkontur des Kerngarns. Bei der Soll-Lage handelt es sich vorzugsweise um eine Soll-Relativlage, insbesondere ebenfalls bezogen auf die Außenkontur.

Die Lage und/oder die Soll-Lage beschreiben vorzugsweise die Konzentrizität des Kernfadens im Kerngarn. Mit anderen Worten kann durch das erfindungsgemäße Verfahren, insbesondere nach einem Spinnprozess und bevorzugt automatisiert, eine Prüfung des Kerngarns hinsichtlich der Konzentrizität vorgenommen werden. Ebenfalls kann durch das erfindungsgemäße Verfahren ggf. die Prüfung hinsichtlich der Abdeckung des Kernfadens vorgenommen werden.

Bei dem Soll-Wert oder den Soll-Werten handelt es sich vorzugsweise um vorbestimmte und/oder vorgegebene und/oder hinterlegte Werte, insbesondere Soll-Werte.

Bei dem Soll-Wert kann es sich vorzugsweise um einen Soll-Werte-Bereich oder eine Soll-Werte-Spanne handeln.

Es ist bevorzugt, wenn der mindestens eine Erfassungswert einem zeitlich gemittelten und/oder einem über die Länge, insbesondere Abschnittslänge, des gesponnenen Kerngarns gemittelten Erfassungswert entspricht. Im Fall einer Abschnittslänge ergibt sich für jeden Abschnitt oder Längenabschnitt des Kerngarns ein entsprechender gemittelter Erfassungswert.

Ebenfalls Gegenstand der Erfindung ist ein Verfahren zum Betrieb eines Spinnstellensystems, wobei das Verfahren die Verfahrensschritte des erfindungsgemäßen Verfahrens zur Bestimmung eines Eigenschaftswerts umfasst, wobei das Spinnstellensystem auf Basis des bestimmten Eigenschaftswerts betrieben wird. Damit bringt das erfindungsgemäße Verfahren zum Betrieb eines Spinnstellensystems die gleichen Vorteile mit sich wie sie bereits für das erfindungsgemäße Verfahren zur Bestimmung eines Eigenschaftswerts eines gesponnenen Kerngarns beschrieben worden sind. Ferner können dadurch verschiedene Spinnparameter in Abhängigkeit des bestimmten Eigenschaftswerts angepasst oder verändert werden, um einen akzeptablen Eigenschaftswert zu erreichen. Mit dem Betrieb ist vorzugsweise eine Regelung und/oder Steuerung des Spinnstellensystems gemeint.

Darüber hinaus ist es bevorzugt, wenn das Verfahren ferner umfasst:
- Initialisieren einer Fortsetzung eines Spinnverfahrens zur Herstellung des Kerngarns, wenn der bestimmte Eigenschaftswert dem akzeptablen Eigenschaftswert entspricht, und/oder
- Initialisieren einer Korrekturmaßnahme, wenn der bestimmte Eigenschaftswert einem inakzeptablen Eigenschaftswert entspricht.

Mit anderen Worten entspricht ein akzeptabler Eigenschaftswert vorzugsweise einem gewünschten Eigenschaftswert, während ein inakzeptabler Eigenschaftswert vorzugsweise einem ungewünschten Eigenschaftswert entspricht. Der inakzeptable Eigenschaftswert führt insbesondere zu Kerngarn, der nicht den Qualitätsanforderungen genügt. Der akzeptable Eigenschaftswert führt insbesondere zu Kerngarn, der den Qualitätsanforderungen genügt.

Darüber hinaus ist es bevorzugt, wenn die Korrekturmaßnahme eine Veränderung, vorzugsweise eine Erhöhung der Zuführung von Fasermaterial, insbesondere Mantelfasern, zum Spinnverfahren und/oder eine Geschwindigkeitsreduzierung des Spinnverfahrens umfasst, um eine Querschnittsdicke der Mantelfaserschicht und/oder eine Abdeckung des Kernfadens mit Mantelfasern zu erhöhen, und/oder dass die Korrekturmaßnahme eine Veränderung der Förderung, insbesondere Erhöhung oder Reduktion der Fördergeschwindigkeit des Kernfadens umfasst, mit der der Kernfaden dem Spinnverfahren zugeführt wird, um die Lage des Kernfadens im Querschnitt des Kerngarns der Soll-Lage anzupassen. Als Korrekturmaßnahmen sind vorzugsweise auch weitere Veränderungen oder Manipulationen von Spinnparametern denkbar.

Darüber hinaus ist es bevorzugt, wenn das Verfahren ferner umfasst:
- Initialisieren einer Wiederholung der Korrekturmaßnahme, wenn der bestimmte Eigenschaftswert nach Durchführung der Korrekturmaßnahme oder der Korrekturmaßnahmen weiterhin dem inakzeptablen Eigenschaftswert entspricht, um einen akzeptablen Eigenschaftswert zu erreichen.

Auf diese Weise wird sichergestellt, dass die Korrekturmaßnahmen wiederholt werden, wenn eine Korrekturmaßnahme nicht ausreichend ist. Vorzugsweise erfolgen die Korrekturmaßnahmen bei jeder Durchführung oder Wiederholung schrittweise, insbesondere in vorgegebenen gleichweiten Schritten. Darunter ist beispielsweise eine schrittweise Reduzierung einer Geschwindigkeit mit jeder Wiederholung zu verstehen. Damit wird eine ruckartige Veränderung der Spinnparameter verhindert.

Darüber hinaus ist es bevorzugt, wenn die Wiederholung mindestens entsprechend einer Mindestanzahl an Wiederholungen und/oder einer Mindestdauer und/oder einer Mindestproduktionslänge des Kerngarns initialisiert wird, um einen akzeptablen Eigenschaftswert zu erreichen, und/oder die Wiederholung maximal entsprechend einer Maximalanzahl an Wiederholungen und/oder einer Maximaldauer und/oder einer Maximalproduktionslänge des Kerngarns initialisiert wird, um einen akzeptablen Eigenschaftswert zu erreichen.

Mit der Mindestanzahl an Wiederholungen und/oder einer Mindestdauer und/oder einer Mindestproduktionslänge wird sichergestellt, dass ausreichend häufig Korrekturmaßnahmen durchgeführt werden können, um einen akzeptablen Eigenschaftswert zu erhalten.

Mit der Maximalanzahl an Wiederholungen und/oder einer Maximaldauer und/oder einer Maximalproduktionslänge wird sichergestellt, dass rechtzeitig sichergestellt werden kann, wenn die Korrekturmaßnahmen nicht den erhofften Erfolg liefern.

Vorzugsweise erfolgen die Korrekturmaßnahmen oder ihre Wiederholungen insbesondere zeitlich, direkt oder beabstandet hintereinander. Vorzugsweise wird mindestens ein aktualisierter Erfassungswert ausgewertet und daraus ein aktualisierter Eigenschaftswert bestimmt, bevor, im Fall, dass der aktualisierte Eigenschaftswert einem inakzeptablen Eigenschaftswert entspricht, eine Wiederholung oder eine neue Korrekturmaßnahme initialisiert wird.

Bei einer Wiederholung einer Korrekturmaßnahme kann es sich im Vergleich zur zuvor initialisierten Korrekturmaßnahme um eine andere oder andersartige Korrekturmaßnahme handeln.

Darüber hinaus ist es bevorzugt, wenn das Verfahren ferner umfasst:
- Initialisieren einer Beendigung oder einer Unterbrechung des Spinnverfahrens, wenn der mindestens eine bestimmte Eigenschaftswert nach Durchführung der Korrekturmaßnahme oder der Korrekturmaßnahmen, insbesondere nach Erreichen der Maximalanzahl an Wiederholungen und/oder der Maximaldauer und/oder der Maximalproduktionslänge des Kerngarns, weiterhin einem inakzeptablen Eigenschaftswert entspricht, und/oder
- Initialisieren eines Signals, insbesondere eines optischen und/oder akustischen Signals, welches spezifisch für den inakzeptablen Eigenschafswert ist, um insbesondere einen Bediener des Spinnstellensystems über die Abweichung zu informieren.

Durch das Signal kann eine Wartung des Spinnstellensystems initialisiert werden, um einen Eigenschaftswert zu erhalten, der einem akzeptablen Eigenschaftswert entspricht.

Das Beenden oder die Unterbrechung ermöglicht die Wartung des Spinnstellensystems. Das Signal kann darüber hinaus über den vorliegenden Eigenschaftswert oder einen Eigenschaftswertverlauf informieren.

Ebenfalls Gegenstand der Erfindung ist eine Vorrichtung zur Datenverarbeitung, die Mittel zur Ausführung einer der erfindungsgemäßen Verfahren umfasst. Damit bringt die erfindungsgemäße Vorrichtung die gleichen Vorteile mit sich wie sie ausführlich mit Bezug auf die erfindungsgemäßen Verfahren beschrieben worden sind.

Bei den Mitteln kann es sich um Programmcode und/oder um konstruktive Elemente und Baugruppen, wie beispielsweise Aktuatoren und/oder Sensorik und/oder Vorrichtungen und/oder Einrichtungen handeln.

Als die Vorrichtung zur Datenverarbeitung kann ein Computer oder eine Datenverarbeitungsvorrichtung angesehen werden. Ferner kann es sich bei der Vorrichtung zur Datenverarbeitung um ein Steuergerät, eine Steuereinheit und/oder eine Auswerteeinheit handeln.

Vorzugsweise kann mittels der Vorrichtung zur Datenverarbeitung der mindestens eine Erfassungswert empfangen werden und/oder der mindestens eine Erfassungswert ausgewertet werden und/oder der Eigenschaftswert bestimmt werden.

Ebenfalls Gegenstand der Erfindung ist eine Fadensensoreinrichtung für ein Spinnstellensystem zur Bestimmung eines Eigenschaftswerts eines Kerngarns, vorzugsweise umfassend die erfindungsgemäße Vorrichtung zur Datenverarbeitung, insbesondere dadurch gekennzeichnet, dass die Fadensensoreinrichtung eine Sensorik umfasst, die dazu ausgebildet ist, mindestens einen Erfassungswert zu erfassen, der für mindestens einen der folgenden Werte spezifisch ist:
- eine Querschnittsdicke des Kerngarns,
- eine Mantelfaserschicht des Kerngarns, insbesondere eine Querschnittsdicke der Mantelfaserschicht und/oder eine Abdeckung des Kernfadens mit Mantelfasern,
- eine Querschnittsdicke des Kernfadens des Kerngarns,
- eine Lage des Kernfadens innerhalb des Querschnitts des Kerngarns.

Ebenfalls Gegenstand der Erfindung ist ein Spinnstellensystem, umfassend eine erfindungsgemäße Fadensensoreinrichtung, aufweisend eine Spinnstellenvorrichtung zur Herstellung des Kerngarns, eine Kernfadenzuführung zum Zuführen des Kernfadens zur Spinnstellenvorrichtung, eine Fasermaterialzuführung zum Zuführen von Mantelfasern zur Spinnstellenvorrichtung, wobei die Fadensensoreinrichtung der Spinnstellenvorrichtung, vorzugsweise in Förderrichtung des Kernfadens, nachgelagert ist, um den Eigenschaftswert des durch die Spinnstellenvorrichtung gesponnenen Kerngarns zu bestimmen.

Unter dem Spinnstellensystem kann vorzugsweise eine Textilmaschine zum Spinnen eines Spinnfadens, insbesondere Kerngarns, oder eine Spinnmaschine zum Spinnen eines Spinnfadens, Kerngarns, verstanden werden. Die Textilmaschine kann zum Rotorspinnen und/oder Luftspinnen und/oder Friktionsspinnen ausgebildet sein. Entsprechend kann das Spinnverfahren ein Open End Spinnen/Rotorspinnen, ein Friktionsspinnen, ein Luftspinnen oder ein anderes Spinnverfahren betreffen/sein.

Dass die Fadensensoreinrichtung der Spinnstellenvorrichtung nachgelagert ist, bedeutet vorzugsweise, dass ein die Spinnstellenvorrichtung oder die Spinnstelle der Spinnstellenvorrichtung verlassendes, gesponnenes Kerngarn die Fadensensoreinrichtung passiert, nachdem es hergestellt wurde.

Die Spinnstellenvorrichtung umfasst vorzugsweise die Spinnstelle, insbesondere in Form eines Rotors, zum Spinnen, vorzugsweise Luftspinnen, des Spinnfadens, insbesondere des Kerngarns.

Mittels der Spinnstellenvorrichtung oder einer Spinnstelle der Spinnstellenvorrichtung kann ein Spinnfaden, insbesondere das Kerngarn, hergestellt werden. Die Spinnstelle kann vorzugsweise einen Rotor umfassen, der dazu ausgebildet ist, einen Kernfaden zusammen mit Rotorfäden, insbesondere Mantelfasern, zu einem Spinnfaden, insbesondere Kerngarn, zu spinnen. Der gesponnene Spinnfaden, insbesondere Kerngarn, wird vorzugsweise nach dessen Herstellung auf eine Spule oder Rolle aufgewickelt und/oder in einer dafür vorgesehenen Kanne untergebracht. Die erfassten Eigenschaftswerte werden der Kanne und/oder der Spule oder Rolle mit dem Spinnfaden, insbesondere Kerngarn, zugeordnet, sodass insbesondere auch zu einem späteren Zeitpunkt Informationen über die bestimmten Eigenschaftswerte zum hergestellten Spinnfaden vorhanden sind.

Das Spinnstellensystem oder Teile des Spinnstellensystems sind vorzugsweise dazu ausgebildet, eines der erfindungsgemäßen Verfahren auszuführen.

Mittels des Spinnstellensystems und/oder dessen Spinnstellenvorrichtung ist Kerngarnspinnen zur Herstellung von Kerngarn ausführbar.

Darüber hinaus ist es bevorzugt, wenn das Spinnstellensystem eine Auswerteeinheit umfasst, die dazu ausgebildet ist, den mindestens einen Erfassungswert auszuwerten, wobei das Auswerten einen Vergleich zwischen dem mindestens einen Erfassungswert und mindestens einem entsprechenden Soll-Wert umfasst, wobei die Auswerteeinheit ferner dazu ausgebildet ist, den Eigenschaftswert des Kerngarns auf Basis der Auswertung zu bestimmen, insbesondere derart, dass ein akzeptabler Eigenschaftswert bestimmt wird, wenn der Erfassungswert dem Soll-Wert entspricht und/oder ein inakzeptabler Eigenschaftswert bestimmt wird, wenn der Erfassungswert vom Soll-Wert abweicht.

Der Soll-Wert ist vorzugsweise für mindestens einen zum Erfassungswert entsprechenden Soll-Wert spezifisch, insbesondere für einen der folgenden Soll-Werte:
- eine Soll-Querschnittsdicke des Kerngarns,
- eine Soll-Mantelfaserschicht des Kerngarns, insbesondere eine Soll-Querschnittsdicke der Mantelfaserschicht, und/oder eine Soll-Abdeckung des Kernfadens mit Mantelfasern,
- eine Soll-Querschnittsdicke des Kernfadens des Kerngarns,
- eine Soll-Lage des Kernfadens innerhalb des Querschnitts des Kerngarns.

Darüber hinaus ist es bevorzugt, wenn das Spinnstellensystem eine Steuereinheit umfasst, die dazu ausgebildet ist, das Spinnstellensystem auf Basis des Eigenschaftswerts zu betreiben, insbesondere derart, dass ein Spinnverfahren zur Herstellung des Kerngarns fortgesetzt wird, wenn der bestimmte Eigenschaftswert dem akzeptablen Eigenschaftswert entspricht, und/oder eine Korrekturmaßnahme durchgeführt wird, wenn der bestimmte Eigenschaftswert einem inakzeptablen Eigenschaftswert entspricht, wobei die Korrekturmaßnahme eine Veränderung, vorzugsweise eine Erhöhung der Zuführung von Fasermaterial, insbesondere Mantelfasern, zum Spinnverfahren und/oder eine Geschwindigkeitsreduzierung des Spinnverfahrens umfasst, um eine Querschnittsdicke der Mantelfaserschicht und/oder eine Abdeckung des Kernfadens mit Mantelfasern zu erhöhen, und/oder die Korrekturmaßnahme eine Veränderung der Förderung, insbesondere Erhöhung oder Reduktion der Fördergeschwindigkeit des Kernfadens umfasst, mit der der Kernfaden dem Spinnverfahren zugeführt wird, um die Lage des Kernfadens im Querschnitt des Kerngarns der Soll-Lage anzupassen.

Bei dem Spinnverfahren handelt es sich vorzugsweise um ein Spinnverfahren zur Herstellung eines Spinnfadens, insbesondere Kerngarns. Durch die Korrekturmaßnahme kann ein möglicher Ausschuss des hergestellten Kerngarns verhindert werden, da lediglich eine Fortsetzung der Produktion, also des Spinnverfahrens erfolgt, wenn das Kerngarn den vorgegebenen Kriterien, also Sollwerten, entspricht.

Mit anderen Worten betrifft das erfindungsgemäße Spinnstellensystem vorzugsweise ein Spinnstellensystem zur Herstellung eines Kerngarns, umfassend eine Fadensensoreinrichtung zur Bestimmung eines Eigenschaftswerts des Kerngarns, wobei die Fadensensoreinrichtung eine Sensorik umfasst, die dazu ausgebildet ist, mindestens einen Erfassungswert zu erfassen, der für mindestens einen der folgenden Werte spezifisch ist:
- eine Querschnittsdicke des Kerngarns,
- eine Mantelfaserschicht des Kerngarns, insbesondere eine Querschnittsdicke der Mantelfaserschicht und/oder eine Abdeckung des Kernfadens mit Mantelfasern,
- eine Querschnittsdicke des Kernfadens des Kerngarns,
- eine Lage des Kernfadens innerhalb des Querschnitts des Kerngarns,
wobei das Spinnstellensystem eine Spinnstellenvorrichtung zur Herstellung des Kerngarns aufweist, wobei das Spinnstellensystem eine Kernfadenzuführung zum Zuführen des Kernfadens zur Spinnstellenvorrichtung aufweist, wobei das Spinnstellensystem eine Fasermaterialzuführung zum Zuführen von Mantelfasern zur Spinnstellenvorrichtung aufweist, wobei die Fadensensoreinrichtung der Spinnstellenvorrichtung, vorzugsweise in Förderrichtung des Kernfadens, nachgelagert ist, um den Eigenschaftswert des durch die Spinnstellenvorrichtung gesponnenen Kerngarns zu bestimmen, wobei das Spinnstellensystem eine Auswerteeinheit umfasst, die dazu ausgebildet ist, den mindestens einen Erfassungswert auszuwerten, wobei das Auswerten einen Vergleich zwischen dem mindestens einen Erfassungswert und mindestens einem entsprechenden Soll-Wert umfasst, wobei die Auswerteeinheit ferner dazu ausgebildet ist, den Eigenschaftswert des Kerngarns auf Basis der Auswertung zu bestimmen, derart, dass ein akzeptabler Eigenschaftswert bestimmt wird, wenn der Erfassungswert dem Soll-Wert entspricht und ein inakzeptabler Eigenschaftswert bestimmt wird, wenn der Erfassungswert vom Soll-Wert abweicht, wobei das Spinnstellensystem eine Steuereinheit umfasst, die dazu ausgebildet ist, das Spinnstellensystem auf Basis des Eigenschaftswerts zu betreiben, derart, dass ein Spinnverfahren zur Herstellung des Kerngarns fortgesetzt wird, wenn der bestimmte Eigenschaftswert dem akzeptablen Eigenschaftswert entspricht, und eine Korrekturmaßnahme durchgeführt wird, wenn der bestimmte Eigenschaftswert einem inakzeptablen Eigenschaftswert entspricht, wobei die Korrekturmaßnahme eine Veränderung, vorzugsweise eine Erhöhung der Zuführung von Fasermaterial, insbesondere Mantelfasern, zum Spinnverfahren und/oder eine Geschwindigkeitsreduzierung des Spinnverfahrens umfasst, um eine Querschnittsdicke der Mantelfaserschicht und/oder eine Abdeckung des Kernfadens mit Mantelfasern zu erhöhen, und/oder die Korrekturmaßnahme eine Veränderung der Förderung, insbesondere Erhöhung oder Reduktion der Fördergeschwindigkeit des Kernfadens umfasst, mit der der Kernfaden dem Spinnverfahren zugeführt wird, um die Lage des Kernfadens im Querschnitt des Kerngarns der Soll-Lage anzupassen, wobei die Steuereinheit ferner dazu ausgebildet ist, das Spinnverfahren des Spinnstellensystems zu beenden, wenn nach einer Maximalanzahl an Wiederholungen und/oder einer oder der Maximaldauer und/oder einer oder der Maximalproduktionslänge des Kerngarns weiterhin ein inakzeptabler Eigenschaftswert bestimmt wird.

Alternativ oder zusätzlich kann das erfindungsgemäße Spinnstellensystem wie folgt beschrieben werden:
Ein Spinnstellensystem, umfassend
- eine Kernfadenzuführung zum Zuführen eines Kernfadens zu einer Spinnstellenvorrichtung,
- eine Fasermaterialzuführung zum Zuführen von Fasermaterial zu der Spinnstellenvorrichtung,
- die Spinnstellenvorrichtung zum Spinnen eines Spinnfadens aus dem Fasermaterial und dem Kernfaden, wobei der Kernfaden den Kern und das Fasermaterial eine an den Kern angrenzende, äußere Mantelfaserschicht bilden,
- eine Fadensensoreinrichtung, welche
   - der Spinnstellenvorrichtung nachgelagert ist,
   - ausgelegt ist, eine Querschnittsdicke des Spinnfadens zu erfassen,
   - ausgelegt ist, eine Querschnittsdicke der Mantelfaserschicht zu erfassen,
   - ausgelegt ist, eine Lage des Kernfadens innerhalb des Spinnfadens zu erfassen,
   - ausgelegt ist, eine Querschnittsdicke des Kernfadens zu erfassen,
- eine Erfassungseinrichtung zum Erfassen einer Produktionslänge des Spinnfadens oder einer Produktionszeit der Spinnstellenvorrichtung,
- eine Auswerteeinrichtung, welche
   - mit der Fadensensoreinrichtung kommunikativ gekoppelt ist,
   - ausgelegt ist, basierend auf den erfassten Daten zu bestimmen,
      - ob der Kernfaden innerhalb des Spinnfadens akzeptabel oder inakzeptabel angeordnet ist,
      - ob die Querschnittsdicke der Mantelfaserschicht für den herzustellenden Spinnfaden mit Kernfaden akzeptabel oder inakzeptabel ist,
- eine Steuereinheit, welche
   - mit der Kernfadenzuführung, der Fasermaterialzuführung, der Spinnstellenvorrichtung, der Erfassungseinrichtung und der Auswerteeinrichtung kommunikativ gekoppelt ist,
   - ausgelegt ist, eine Zuführgeschwindigkeit der Kernfadenzuführung zur Lagekorrektur des Kernfadens zur akzeptablen Anordnung innerhalb des herzustellenden Spinnfadens zu steuern,
   - ausgelegt ist, eine Zuführgeschwindigkeit der Fasermaterialzuführung zur Korrektur der Querschnittsdicke der Mantelfaserschicht innerhalb des herzustellenden Spinnfadens in einen akzeptablen Bereich zu steuern,
   - ausgelegt ist, definierte Spinnparameter der Spinneinrichtung zur Korrektur der Querschnittsdicke der Mantelfaserschicht innerhalb des herzustellenden Spinnfadens in einen akzeptablen Bereich zu steuern,
   - ausgelegt ist, einen produktiven Betrieb des Spinnstellensystems zu sperren, wenn eine der Korrekturen nach Ablauf einer definierten Produktionszeit oder Produktionslänge inakzeptabel bestimmt ist, wobei der produktive Betrieb ein solcher Betrieb ist, bei welchem die Spinnstellenvorrichtung den Spinnfaden herstellt.

Einzelne oder mehrere der mit Aufzählungszeichen oder Spiegelstrichen angeführten Merkmale der direkt obenstehend beschriebenen Ausführungsform können optional sein.

Ebenfalls Gegenstand der Erfindung ist ein Computerprogramm, insbesondere Computerprogrammprodukt, welches Befehle umfasst, die bei der Ausführung des Computerprogramms durch einen Computer diesen veranlassen, eines der erfindungsgemäßen Verfahren auszuführen.

Damit bringt das erfindungsgemäße Computerprogramm, insbesondere Computerprogrammprodukt, die gleichen Vorteile mit sich wie sie ausführlich mit Bezug auf ein erfindungsgemäßes Verfahren beschrieben worden sind.

Als der Computer kann die Vorrichtung zur Datenverarbeitung, eine Datenverarbeitungsvorrichtung, bspw. eine der erfindungsgemäßen Vorrichtungen, vorgesehen sein, welche das Computerprogramm ausführt. Der Computer kann wenigstens einen Prozessor zur Ausführung des Computerprogramms aufweisen. Auch kann ein nicht-flüchtiger Datenspeicher vorgesehen sein, in welchem das Computerprogramm hinterlegt und von welchem das Computerprogramm durch den Prozessor zur Ausführung ausgelesen werden kann.

Ebenfalls ist es denkbar, dass der Computer zumindest einen integrierten Schaltkreis wie einen Mikroprozessor oder eine Anwendungsspezifische integrierte Schaltung (ASIC) oder ein Anwendungsspezifisches Standardprodukt (ASSP) oder einen digitalen Signalprozessor (DSP) oder einen Field Programmable Gate Array (FPGA) oder dergleichen umfasst. Der Computer kann ferner wenigstens eine Schnittstelle zum Datenaustausch, z. B. eine Ethernet-Schnittstelle oder eine Schnittstelle für LAN (Local Area Network) oder WLAN (Wireless Local Area Network) oder System-on-a-Chip (SoC) oder eine andere Funkschnittstelle wie Bluetooth oder Nahfeldkommunikation (NFC) aufweisen. Ferner kann der Computer als ein oder mehrere Steuergeräte, d. h. auch als ein System aus Steuergeräten, ausgeführt sein. Der Computer kann bspw. auch in einer Cloud und/oder als ein Server vorgesehen sein, um über die Schnittstelle die Datenverarbeitung für eine lokale Anwendung zur Verfügung zu stellen. Auch ist es möglich, dass der Computer als ein mobiles Gerät, wie ein Smartphone, ausgeführt ist.

Das Computerprogramm kann auch bereitgestellt werden, indem es auf einem nicht-flüchtigen Speicher oder einem computerlesbaren Medium abgespeichert ist.

Außerdem ist ein computerlesbares Medium denkbar, auf dem das erfindungsgemäße Computerprogramm abgespeichert ist.

Damit bringt das computerlesbare Medium die gleichen Vorteile mit sich wie sie ausführlich mit Bezug auf ein erfindungsgemäßes Computerprogramm beschrieben worden sind.

Ebenfalls Gegenstand der Erfindung kann ein computerlesbares Speichermedium sein, welches das erfindungsgemäße Computerprogramm umfasst. Das Speichermedium ist bspw. als ein Datenspeicher wie eine Festplatte und/oder ein nicht-flüchtiger Speicher und/oder eine Speicherkarte ausgebildet. Das Speichermedium kann z. B. in den Computer integriert sein.

Darüber hinaus kann eines der erfindungsgemäßen Verfahren, insbesondere vollständig oder teilweise, als ein computerimplementiertes Verfahren und/oder als ein automatisiertes Verfahren ausgeführt sein.

### Figurenbeschreibung

Weitere Vorteile, Merkmale und Einzelheiten der Erfindung ergeben sich aus der nachfolgenden Beschreibung, in der unter Bezugnahme auf die Zeichnungen Ausführungsbeispiele der Erfindung im Einzelnen beschrieben sind. Dabei können die in den Ansprüchen und in der Beschreibung erwähnten Merkmale jeweils einzeln für sich oder in beliebiger Kombination erfindungswesentlich sein.

Es zeigen:
- Fig. 1: ein Ablaufdiagramm eines Ausführungsbeispiels eines erfindungsgemäßen Verfahrens,
- Fig. 2: eine schematische Darstellung eines Ausführungsbeispiels eines erfindungsgemäßen Spinnstellensystems,
- Fig. 3a: eine schematische Darstellung der Lage eines Kernfadens in einem Kerngarn, und
- Fig. 3b: eine weitere schematische Darstellung der Lage eines Kernfadens in einem Kerngarn.

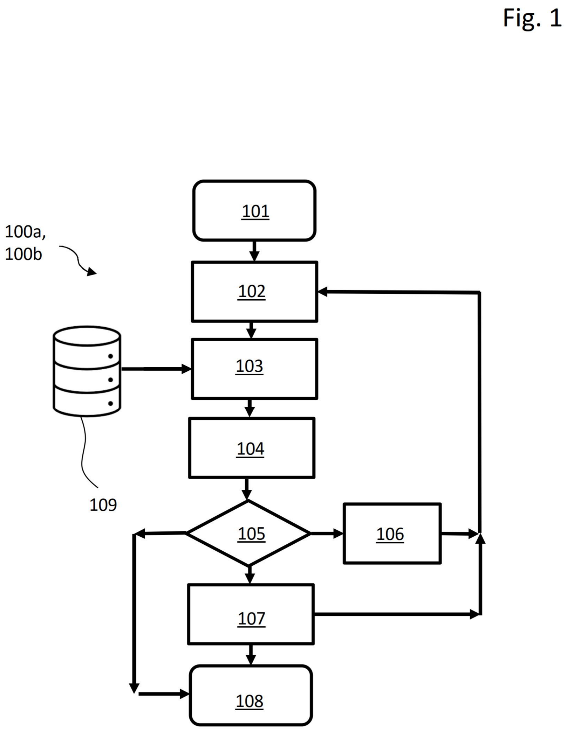

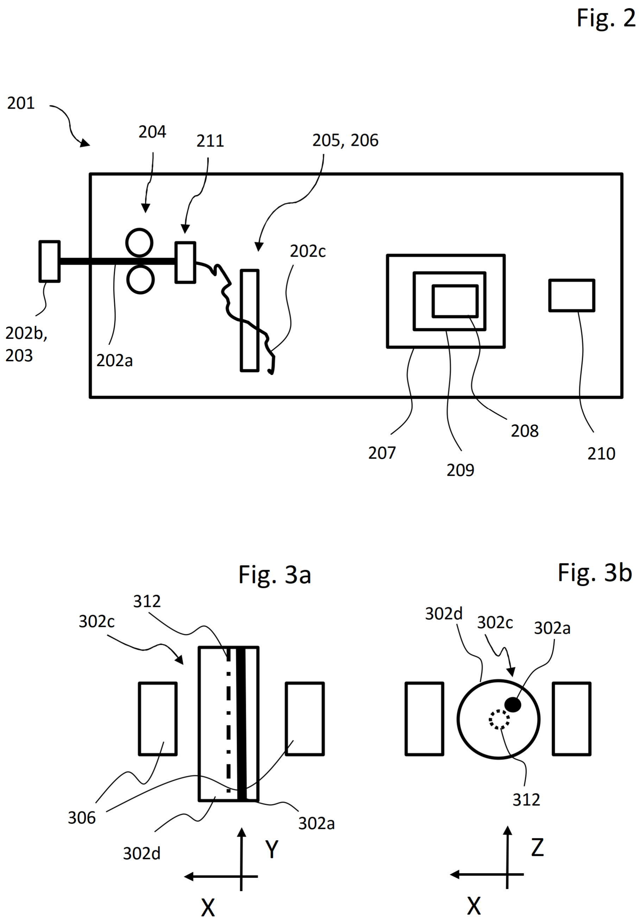

Die Figur 1 zeigt ein Ablaufdiagramm eines Ausführungsbeispiels eines erfindungsgemäßen Verfahrens 100b zum Betrieb eines Spinnstellensystems 201, welches die Verfahrensschritte eines erfindungsgemäßen Verfahrens 100a zur Bestimmung eines Eigenschaftswerts eines gesponnenen Kerngarns 202c, 302c umfasst, wobei das Kerngarn 202c, 302c einen Kernfaden 202a, 302a und Mantelfasern 302d umfasst, die den Kernfaden 202a, 302a umgeben (Fig. 3a, 3b). Der erste Verfahrensschritt 101 erfasst ein Initialisieren des erfindungsgemäßen Verfahrens 100b zum Betrieb eines Spinnstellensystems 201, welches zeitgleich mit dem Initialisieren eines Herstellverfahrens von Kerngarn 202c, 302c einhergehen kann. Als zweiter Verfahrensschritt 102 erfolgt das Empfangen von mindestens einem Erfassungswert. Der mindestens eine Erfassungswert ist für mindestens einen der folgenden Werte spezifisch:
- eine Querschnittsdicke des Kerngarns 202c, 302c,
- eine aus den Mantelfasern 302d gebildete Mantelfaserschicht des Kerngarns 202c, 302c, insbesondere eine Querschnittsdicke der Mantelfaserschicht und/oder eine Abdeckung des Kernfadens 202a, 302a mit Mantelfasern 302d,
- eine Querschnittsdicke des Kernfadens 202a, 302a des Kerngarns 202c, 302c,
- eine Lage des Kernfadens 202a, 302a innerhalb des Querschnitts des Kerngarns 202c, 302c.

Als nächstes erfolgt ein dritter Verfahrensschritt 103, der ein Auswerten des mindestens einen Erfassungswerts umfasst, wobei das Auswerten einen Vergleich zwischen dem mindestens einen Erfassungswert und mindestens einem in einer Datenbank eines Speichers 109 hinterlegten Soll-Wert umfasst. Als vierter Verfahrensschritt 104 erfolgt ein Bestimmen des Eigenschaftswerts des Kerngarns 202c, 302c auf Basis der Auswertung. Es wird ein akzeptabler Eigenschaftswert bestimmt, wenn der Erfassungswert einem Soll-Wert entspricht und es wird ein inakzeptabler Eigenschaftswert bestimmt, wenn der Erfassungswert vom Soll-Wert abweicht. Als fünfter Verfahrensschritt 105 wird entweder ein sechster Verfahrensschritt 106 eingeleitet, wenn der bestimmte Eigenschaftswert einem inakzeptablen Eigenschaftswert entspricht. Der sechste Verfahrensschritt 106 umfasst ein Initialisieren einer Korrekturmaßnahme. Die Korrekturmaßnahme kann beispielsweise eine Erhöhung der Zuführung von Mantelfasern 302d zum Spinnverfahren und eine Geschwindigkeitsreduzierung des Spinnverfahrens umfassen, um eine Querschnittsdicke der Mantelfaserschicht und eine Abdeckung des Kernfadens 202a, 302a mit Mantelfasern 302d zu erhöhen. Die Korrekturmaßnahme kann zusätzlich oder alternativ eine Erhöhung oder Reduktion der Fördergeschwindigkeit des Kernfadens 202a, 302a umfassen, mit der der Kernfaden 202a, 302a dem Spinnverfahren zugeführt wird, um die Lage des Kernfadens 202a, 302a im Querschnitt des Kerngarns 202c, 302c der Soll-Lage 312 anzupassen. Daraufhin wird das Verfahren 100b, beginnend mit dem zweiten Verfahrensschritt 102, wiederholt. Eine derartige Wiederholung und das Durchführen einer Korrekturmaßnahme kann entsprechend einer vorgegebenen Maximalanzahl an Wiederholungen begrenzt sein. Wenn die Maximalanzahl an Wiederholungen von Korrekturmaßnahmen erfolgt ist, kann direkt nach dem fünften Verfahrensschritt 105 der achte Verfahrensschritt 108 erfolgen, der ein Beenden oder Unterbrechen des Spinnverfahrens und des Verfahrens vorsieht, um beispielsweise eine Wartung an dem Spinnstellensystem 201, mit dem das Spinnverfahren ausgeübt worden ist, vorzunehmen. Wenn es sich bei dem mindestens einen Eigenschaftswert jedoch um einen akzeptablen Eigenschaftswert handelt, wird im Rahmen des fünften Verfahrensschritts 105 der siebte Verfahrensschritt 107 initialisiert, der ein Fortsetzen des Spinnvorgangs umfasst. Optional kann das Verfahren 100b zum Betrieb eines Spinnstellensystems 201 in Echtzeit betrieben werden und über die gesamte Dauer des Spinnvorgangs andauern, was bedeutet, dass auf den siebten Verfahrensschritt 107 wieder ein Empfang eines aktuellen Erfassungswerts erfolgt. Wenn im siebten Verfahrensschritt 107 jedoch festgestellt werden sollte, dass kein Material mehr zum Spinnen vorhanden ist, wird der achte Verfahrensschritt 108 eingeleitet, der ein Beenden oder Unterbrechen des Spinnverfahrens umfasst, um das Material aufzufüllen.

Die Figur 2 zeigt eine schematische Darstellung eines Ausführungsbeispiels eines erfindungsgemäßen Spinnstellensystems 201. Das Spinnstellensystems 201 umfasst eine Halterung 203 für eine Kernfadenspule 202b. Von der Kernfadenspule 202b kann mittels einer Fördervorrichtung 204 ein Kernfaden 202a abgezogen und zu einer Spinnstellenvorrichtung 211 gefördert werden, wo der Kernfaden 202a zusammen mit Mantelfasern 302d zu einem Kerngarn 202c gesponnen werden kann. Mit anderen Worten handelt es sich bei der Fördervorrichtung 204 um eine Kernfadenzuführung 204. Eine Fasermaterialzuführung zum Zuführen von Mantelfasern 302d zur Spinnstellenvorrichtung 211 ist zur Vereinfachung der Darstellung nicht dargestellt, auch wenn das Spinnstellensystem 201 eine solche Fasermaterialzuführung umfasst. Das gesponnene Kerngarn 202c wird mittels einer Fadensensoreinrichtung 205, die eine Sensorik 206 aufweist, überprüft, indem Erfassungswerte erfasst werden. Diese Erfassungswerte werden auf einer Ausführungsform der erfindungsgemäßen Vorrichtung 207 zur Datenverarbeitung, die als Computer 207 und Steuereinheit 207 ausgebildet ist, ausgewertet und der Eigenschaftswert des Kerngarns 202c bestimmt. Hierfür weist die Steuereinheit 207 ein computerlesbares Medium 209 auf, auf welchem eine Ausführungsform des erfindungsgemäßen Computerprogramms 208 gespeichert ist. Das computerlesbare Medium 209 ist vorzugsweise als nicht-flüchtiger Speicher 109 ausgebildet. Das Computerprogramm 208 umfasst Befehle, die bei der Ausführung des Computerprogramms 208 durch einen Computer 207 diesen veranlassen, das erfindungsgemäße Verfahren zum Betrieb des Spinnstellensystems 201 auszuführen. Außerdem umfasst das Spinnstellensystem 201 einen Signalgeber 210, der dazu ausgebildet ist, ein akustisches und/oder optisches Signal an einen Bediener des Spinnstellensystems 201 auszugeben, wenn eine Maximalzahl an Wiederholungen von Korrekturmaßnahmen erfolglos geblieben ist. Das Signal ist dabei charakteristisch für den inakzeptablen Eigenschaftswert.

Die Figur 3a zeigt eine schematische Darstellung der Lage eines Kernfadens 302a in einem Kerngarn 302c. Es handelt sich bei der Ansicht um einen Längsschnitt durch das Kerngarn 302c in der X-Y-Ebene, wobei das Kerngarn 302c in Y-Richtung gefördert wird. Mittels der Sensorik 306 lässt sich die Lage des Kernfadens 302a, welcher von Mantelfasern 302d umgeben ist, erfassen. Dargestellt ist eine Lage des Kernfadens 302a außerhalb der Soll-Lage 312 des Kernfadens 302a, was zu einer Bestimmung eines inakzeptablen Eigenschaftswerts des Kerngarns 302c führen würde.

Die Figur 3b zeigt eine schematische Darstellung der Lage eines Kernfadens 302a in einem Kerngarn 302c. Es handelt sich um die Anordnung aus Figur 3a. Es ist jedoch ein Querschnitt, senkrecht zur Förderrichtung des Kerngarns 302c, also in der X-Z-Ebene dargestellt, was die Abweichung der Lage des Kernfadens 302a von einer Soll-Lage 312, insbesondere einer optimalen mittigen, also konzentrischen, Position innerhalb der Mantelfasern 302d, verdeutlicht.

Ausführungsvarianten der Erfindung gehen von einer Situation aus, bei welcher eine Prüfung eines Kerngarns 202c, 302c nach dem Spinnprozess hinsichtlich Kriterien wie Abdeckung und Konzentrizität des Kernfadens 202a, 302a durchgeführt wird. Hierbei interagieren insbesondere die Prozesse mit den Steuerungen der Maschine, Spinnstelle und/oder Anlage bzw. kompletten Spinnerei, wobei die Prozesse automatisiert sein können. Nachdem ein Kerngarn 202c, 302c produziert worden ist, stellt sich häufig die Frage, wie der Kernfaden 202a, 302a von Mantelfasern 302d abgedeckt und wie zentrisch der Kernfaden 202a, 302a im Kerngarn 202c, 302c positioniert ist.

Gemäß Ausführungsvarianten der Erfindung ist vorgesehen, dass - nachdem das Kerngarn 202c, 302c den Spinnprozess verlassen hat - das Kerngarn 202c, 302c eine Sensorik 206, 306 durchläuft. Mithilfe der Sensorik 206, 306 kann ein geometrisches Bild vom Ist-Zustand des Kerngarns 202c, 302c ermittelt werden. Der Ist-Zustand kann sodann mit einem Sollwert verglichen werden. Dabei kann die Mittenlage des Kernfadens 202a, 302a und die Abdeckung mit Mantelfasern 302d verglichen werden. Sollten Abweichungen vom Soll vorhanden sein, können ggf. Maßnahmen zur Korrektur eingeleitet werden.

Sollte sich der Kernfaden 202a, 302a außerhalb der Mitte des Kerngarns 202c, 302c befinden, kann eine Korrektur durch eine Geschwindigkeitsänderung der Kernfadenzuführung 204 erfolgen. Hierzu können die Geschwindigkeiten vorgelagerter Kernfaden-Förderwerke angepasst werden. Befinden sich zu wenige Mantelfasern 302d im Kerngarn 202c, 302c, kann dies durch eine Erhöhung der Einspeisung verändert werden. Ebenfalls kann eine Erhöhung der Zuführgeschwindigkeit von Fasermaterial und z. B. der Drehzahl der Auflösewalze und/oder eine Reduzierung der Drehzahl des garnbildenden Prozesses in Betracht gezogen werden. Nachdem die Änderungen der Parameter erfolgt sind, kann weiterhin das Kerngarn 202c, 302c über die Sensorik 206, 306 beobachtet werden. Befindet sich der Kernfaden 202a, 302a zentrisch und ist eine ausreichende Abdeckung vorhanden, kann der Produktionsprozess weiterlaufen. Sind die Anforderungen nicht erfüllt, kann es vorgesehen sein, dass die vorgelagerten Prozesse weiter verändert werden. Werden die Vorgaben nach einer vorgegebenen Produktionslänge nicht erreicht, kann die Produktionsstelle in Störung gesetzt werden.

### Bezugszeichenliste

- 100a: Verfahren
- 100b: Verfahren
- 101: erster Verfahrensschritt
- 102: zweiter Verfahrensschritt
- 103: dritter Verfahrensschritt
- 104: vierter Verfahrensschritt
- 105: fünfter Verfahrensschritt
- 106: sechster Verfahrensschritt
- 107: siebter Verfahrensschritt
- 108: achter Verfahrensschritt
- 109: Speicher

- 201: Spinnstellensystem
- 202a: Kernfaden
- 202b: Kernfadenspule
- 202c: Kerngarn
- 203: Halterung
- 204: Fördervorrichtung, Kernfadenzuführung
- 205: Fadensensoreinrichtung
- 206: Sensorik
- 207: Vorrichtung, Computer, Steuereinheit
- 208: Computerprogramm
- 209: computerlesbares Medium
- 210: Signalgeber
- 211: Spinnstellenvorrichtung

- 302a: Kernfaden
- 302c: Kerngarn
- 302d: Mantelfasern
- 306: Sensorik
- 312: Soll-Lage

## Patentansprüche

1. Verfahren (100a) zur Bestimmung eines Eigenschaftswerts eines gesponnenen Kerngarns (202c, 302c), wobei das Kerngarn (202c, 302c) einen Kernfaden (202a, 302a) und Mantelfasern (302d) umfasst, die den Kernfaden (202a, 302a) umgeben, umfassend:
- Empfangen von mindestens einem Erfassungswert, wobei der mindestens eine Erfassungswert vorzugsweise für einen Querschnitt des Kerngarns (202c, 302c) spezifisch ist,
- Auswerten des mindestens einen Erfassungswerts, wobei das Auswerten einen Vergleich zwischen dem mindestens einen Erfassungswert und mindestens einem Soll-Wert umfasst,
- Bestimmen des Eigenschaftswerts des Kerngarns (202c, 302c) auf Basis der Auswertung, insbesondere eines akzeptablen Eigenschaftswerts, wenn der Erfassungswert einem Soll-Wert entspricht und/oder eines inakzeptablen Eigenschaftswerts, wenn der Erfassungswert vom Soll-Wert abweicht.

2. Verfahren (100a) gemäß Anspruch 1, wobei der mindestens eine Erfassungswert für mindestens einen der folgenden Werte spezifisch ist:
- eine Querschnittsdicke des Kerngarns (202c, 302c),
- eine aus den Mantelfasern (302d) gebildete Mantelfaserschicht des Kerngarns (202c, 302c), insbesondere eine Querschnittsdicke der Mantelfaserschicht und/oder eine Abdeckung des Kernfadens (202a, 302a) mit Mantelfasern (302d),
- eine Querschnittsdicke des Kernfadens (202a, 302a) des Kerngarns (202c, 302c),
- eine Lage des Kernfadens (202a, 302a) innerhalb des Querschnitts des Kerngarns (202c, 302c),
und wobei der Soll-Wert vorzugsweise für mindestens einen zum Erfassungswert entsprechenden Soll-Wert spezifisch ist, insbesondere für einen der folgenden Soll-Werte:
- eine Soll-Querschnittsdicke des Kerngarns (202c, 302c),
- eine Soll-Mantelfaserschicht des Kerngarns (202c, 302c), insbesondere eine Soll-Querschnittsdicke der Mantelfaserschicht, und/oder eine Soll-Abdeckung des Kernfadens (202a, 302a) mit Mantelfasern (302d),
- eine Soll-Querschnittsdicke des Kernfadens (202a, 302a) des Kerngarns (202c, 302c),
- eine Soll-Lage (312) des Kernfadens (202a, 302a) innerhalb des Querschnitts des Kerngarns (202c, 302c).

3. Verfahren (100b) zum Betrieb eines Spinnstellensystems (201), wobei das Verfahren (100b) die Verfahrensschritte nach Anspruch 1 oder 2 umfasst, **dadurch gekennzeichnet, dass** das Spinnstellensystem (201) auf Basis des bestimmten Eigenschaftswerts betrieben wird.

4. Verfahren (100b) nach Anspruch 3, ferner umfassend:
- Initialisieren einer Fortsetzung eines Spinnverfahrens zur Herstellung des Kerngarns (202c, 302c), wenn der bestimmte Eigenschaftswert dem akzeptablen Eigenschaftswert entspricht, und/oder
- Initialisieren einer Korrekturmaßnahme, wenn der bestimmte Eigenschaftswert einem inakzeptablen Eigenschaftswert entspricht.

5. Verfahren (100b) nach Anspruch 3 oder 4, **dadurch gekennzeichnet, dass** die Korrekturmaßnahme eine Veränderung, vorzugsweise eine Erhöhung der Zuführung von Fasermaterial, insbesondere Mantelfasern (302d), zum Spinnverfahren und/oder eine Geschwindigkeitsreduzierung des Spinnverfahrens umfasst, um eine Querschnittsdicke der Mantelfaserschicht und/oder eine Abdeckung des Kernfadens (202a, 302a) mit Mantelfasern (302d) zu erhöhen, und/oder dass die Korrekturmaßnahme eine Veränderung der Förderung, insbesondere Erhöhung oder Reduktion der Fördergeschwindigkeit des Kernfadens (202a, 302a) umfasst, mit der der Kernfaden (202a, 302a) dem Spinnverfahren zugeführt wird, um die Lage des Kernfadens (202a, 302a) im Querschnitt des Kerngarns (202c, 302c) der Soll-Lage (312) anzupassen.

6. Verfahren (100b) nach einem der Ansprüche 3 bis 5, ferner umfassend:
- Initialisieren einer Wiederholung der Korrekturmaßnahme, wenn der bestimmte Eigenschaftswert nach Durchführung der Korrekturmaßnahme oder der Korrekturmaßnahmen weiterhin dem inakzeptablen Eigenschaftswert entspricht, um einen akzeptablen Eigenschaftswert zu erreichen.

7. Verfahren (100b) nach Anspruch 6, **dadurch gekennzeichnet, dass** die Wiederholung mindestens entsprechend einer Mindestanzahl an Wiederholungen und/oder einer Mindestdauer und/oder einer Mindestproduktionslänge des Kerngarns (202c, 302c) initialisiert wird, um einen akzeptablen Eigenschaftswert zu erreichen, und/oder die Wiederholung maximal entsprechend einer Maximalanzahl an Wiederholungen und/oder einer Maximaldauer und/oder einer Maximalproduktionslänge des Kerngarns (202c, 302c) initialisiert wird, um einen akzeptablen Eigenschaftswert zu erreichen.

8. Verfahren (100b) nach einem der vorhergehenden Ansprüche, ferner umfassend:
- Initialisieren einer Beendigung oder einer Unterbrechung des Spinnverfahrens, wenn der mindestens eine bestimmte Eigenschaftswert nach Durchführung der Korrekturmaßnahme oder der Korrekturmaßnahmen, insbesondere nach Erreichen der Maximalanzahl an Wiederholungen und/oder der Maximaldauer und/oder der Maximalproduktionslänge des Kerngarns (202c, 302c), weiterhin einem inakzeptablen Eigenschaftswert entspricht, und/oder
- Initialisieren eines Signals, insbesondere eines optischen und/oder akustischen Signals, welches spezifisch für den inakzeptablen Eigenschafswert ist, um insbesondere einen Bediener des Spinnstellensystems (201) über die Abweichung zu informieren.

9. Vorrichtung (207) zur Datenverarbeitung, die Mittel zur Ausführung eines Verfahrens (100a, 100b) nach einem der Ansprüche 1 bis 8 umfasst.

10. Fadensensoreinrichtung (205) für ein Spinnstellensystem (201) zur Bestimmung eines Eigenschaftswerts eines Kerngarns (202c, 302c), umfassend die Vorrichtung (207) gemäß Anspruch 9, insbesondere **dadurch gekennzeichnet, dass** die Fadensensoreinrichtung (205) eine Sensorik (206, 306) umfasst, die dazu ausgebildet ist, den mindestens einen Erfassungswert zu erfassen, der für mindestens einen der folgenden Werte spezifisch ist:
- eine Querschnittsdicke des Kerngarns (202c, 302c),
- eine aus den Mantelfasern (302d) gebildete Mantelfaserschicht des Kerngarns (202c, 302c), insbesondere eine Querschnittsdicke der Mantelfaserschicht und/oder eine Abdeckung des Kernfadens (202a, 302a) mit den Mantelfasern (302d),
- eine Querschnittsdicke des Kernfadens (202a, 302a) des Kerngarns (202c, 302c),
- eine Lage des Kernfadens (202a, 302a) innerhalb des Querschnitts des Kerngarns (202c, 302c).

11. Spinnstellensystem (201), umfassend eine Fadensensoreinrichtung (205) gemäß Anspruch 10, aufweisend eine Spinnstellenvorrichtung (211) zur Herstellung des Kerngarns (202c, 302c), eine Kernfadenzuführung (204) zum Zuführen des Kernfadens (202a, 302a) zur Spinnstellenvorrichtung (211), eine Fasermaterialzuführung zum Zuführen von Mantelfasern (302d) zur Spinnstellenvorrichtung (211), wobei die Fadensensoreinrichtung (205) der Spinnstellenvorrichtung (211), vorzugsweise in Förderrichtung des Kernfadens (202a, 302a), nachgelagert ist, um den Eigenschaftswert des durch die Spinnstellenvorrichtung (211) gesponnenen Kerngarns (202c, 302c) zu bestimmen.

12. Spinnstellensystem (201) gemäß Anspruch 11, **dadurch gekennzeichnet, dass** das Spinnstellensystem (201) eine Auswerteeinheit umfasst, die dazu ausgebildet ist, den mindestens einen Erfassungswert auszuwerten, wobei das Auswerten einen Vergleich zwischen dem mindestens einen Erfassungswert und mindestens einem entsprechenden Soll-Wert umfasst, dass die Auswerteeinheit ferner dazu ausgebildet ist, den Eigenschaftswert des Kerngarns (202c, 302c) auf Basis der Auswertung zu bestimmen, insbesondere derart, dass ein akzeptabler Eigenschaftswert bestimmt wird, wenn der Erfassungswert dem Soll-Wert entspricht und/oder ein inakzeptabler Eigenschaftswert bestimmt wird, wenn der Erfassungswert vom Soll-Wert abweicht.

13. Spinnstellensystem (201) gemäß Anspruch 11 oder 12, **dadurch gekennzeichnet, dass** das Spinnstellensystem (201) eine Steuereinheit (207) umfasst, die dazu ausgebildet ist, das Spinnstellensystem (201) auf Basis des Eigenschaftswerts zu betreiben, insbesondere derart, dass ein Spinnverfahren zur Herstellung des Kerngarns (202c, 302c) fortgesetzt wird, wenn der bestimmte Eigenschaftswert dem akzeptablen Eigenschaftswert entspricht, und/oder eine Korrekturmaßnahme durchgeführt wird, wenn der bestimmte Eigenschaftswert einem inakzeptablen Eigenschaftswert entspricht, wobei die Korrekturmaßnahme eine Veränderung, vorzugsweise eine Erhöhung der Zuführung von Fasermaterial, insbesondere Mantelfasern (302d), zum Spinnverfahren und/oder eine Geschwindigkeitsreduzierung des Spinnverfahrens umfasst, um eine Querschnittsdicke der Mantelfaserschicht und/oder eine Abdeckung des Kernfadens (202a, 302a) mit Mantelfasern (302d) zu erhöhen, und/oder die Korrekturmaßnahme eine Veränderung der Förderung, insbesondere Erhöhung oder Reduktion der Fördergeschwindigkeit des Kernfadens (202a, 302a) umfasst, mit der der Kernfaden (202a, 302a) dem Spinnverfahren zugeführt wird, um die Lage des Kernfadens (202a, 302a) im Querschnitt des Kerngarns (202c, 302c) der Soll-Lage (312) anzupassen.

14. Spinnstellensystem (201) zur Herstellung eines Kerngarns (202c, 302c), umfassend eine Fadensensoreinrichtung (205) nach Anspruch 10 zur Bestimmung eines Eigenschaftswerts des Kerngarns (202c, 302c), wobei die Fadensensoreinrichtung (205) eine Sensorik (206, 306) umfasst, die dazu ausgebildet ist, mindestens einen Erfassungswert zu erfassen, der für mindestens einen der folgenden Werte spezifisch ist:
- eine Querschnittsdicke des Kerngarns (202c, 302c),
- eine Mantelfaserschicht des Kerngarns (202c, 302c), insbesondere eine Querschnittsdicke der Mantelfaserschicht und/oder eine Abdeckung des Kernfadens (202a, 302a) mit Mantelfasern (302d),
- eine Querschnittsdicke des Kernfadens (202a, 302a) des Kerngarns (202c, 302c),
- eine Lage des Kernfadens (202a, 302a) innerhalb des Querschnitts des Kerngarns (202c, 302c),
wobei das Spinnstellensystem (201) eine Spinnstellenvorrichtung (211) zur Herstellung des Kerngarns (202c, 302c) aufweist, wobei das Spinnstellensystem (201) eine Kernfadenzuführung (204) zum Zuführen des Kernfadens (202a, 302a) zur Spinnstellenvorrichtung (211) aufweist, wobei das Spinnstellensystem (201) eine Fasermaterialzuführung zum Zuführen von Mantelfasern (302d) zur Spinnstellenvorrichtung (211) aufweist, wobei die Fadensensoreinrichtung (205) der Spinnstellenvorrichtung (211), vorzugsweise in Förderrichtung des Kernfadens (202a, 302a), nachgelagert ist, um den Eigenschaftswert des durch die Spinnstellenvorrichtung (211) gesponnenen Kerngarns (202c, 302c) zu bestimmen, wobei das Spinnstellensystem (201) eine Auswerteeinheit umfasst, die dazu ausgebildet ist, den mindestens einen Erfassungswert auszuwerten, wobei das Auswerten einen Vergleich zwischen dem mindestens einen Erfassungswert und mindestens einem entsprechenden Soll-Wert umfasst, wobei die Auswerteeinheit ferner dazu ausgebildet ist, den Eigenschaftswert des Kerngarns (202c, 302c) auf Basis der Auswertung zu bestimmen, derart, dass ein akzeptabler Eigenschaftswert bestimmt wird, wenn der Erfassungswert dem Soll-Wert entspricht und ein inakzeptabler Eigenschaftswert bestimmt wird, wenn der Erfassungswert vom Soll-Wert abweicht, wobei das Spinnstellensystem (201) eine Steuereinheit (207) umfasst, die dazu ausgebildet ist, das Spinnstellensystem (201) auf Basis des Eigenschaftswerts zu betreiben, derart, dass ein Spinnverfahren zur Herstellung des Kerngarns (202c, 302c) fortgesetzt wird, wenn der bestimmte Eigenschaftswert dem akzeptablen Eigenschaftswert entspricht, und eine Korrekturmaßnahme durchgeführt wird, wenn der bestimmte Eigenschaftswert einem inakzeptablen Eigenschaftswert entspricht, wobei die Korrekturmaßnahme eine Veränderung, vorzugsweise eine Erhöhung der Zuführung von Fasermaterial, insbesondere Mantelfasern (302d), zum Spinnverfahren und/oder eine Geschwindigkeitsreduzierung des Spinnverfahrens umfasst, um eine Querschnittsdicke der Mantelfaserschicht und/oder eine Abdeckung des Kernfadens (202a, 302a) mit Mantelfasern (302d) zu erhöhen, und/oder die Korrekturmaßnahme eine Veränderung der Förderung, insbesondere Erhöhung oder Reduktion der Fördergeschwindigkeit des Kernfadens (202a, 302a) umfasst, mit der der Kernfaden (202a, 302a) dem Spinnverfahren zugeführt wird, um die Lage des Kernfadens (202a, 302a) im Querschnitt des Kerngarns (202c, 302c) der Soll-Lage (312) anzupassen, wobei die Steuereinheit (207) ferner dazu ausgebildet ist, das Spinnverfahren des Spinnstellensystems (201) zu beenden, wenn nach einer Maximalanzahl an Wiederholungen und/oder einer Maximaldauer und/oder einer Maximalproduktionslänge des Kerngarns (202c, 302c) weiterhin ein inakzeptabler Eigenschaftswert bestimmt wird.

15. Computerprogramm (208), umfassend Befehle, die bei der Ausführung des Computerprogramms (208) durch einen Computer (207) diesen veranlassen, ein Verfahren (100a, 100b) nach einem der Ansprüche 1 bis 8 auszuführen.
